(19) Europäisches Patentamt
European Patent Office
Office européen des brevets

(11) Publication number: **0 080 283**
**B1**

(12) # EUROPEAN PATENT SPECIFICATION

(45) Date of publication of patent specification: **29.01.86**

(51) Int. Cl.$^4$: **C 07 K 5/08,** A 61 K 31/02, A 61 K 37/02

(21) Application number: **82305879.7**

(22) Date of filing: **05.11.82**

(54) **N-carboxyalkylproline-containing tripeptides.**

(30) Priority: **06.11.81 US 318721**

(43) Date of publication of application:
**01.06.83 Bulletin 83/22**

(45) Publication of the grant of the patent:
**29.01.86 Bulletin 86/05**

(84) Designated Contracting States:
**AT BE CH DE FR GB IT LI LU NL SE**

(56) References cited:
**EP-A-0 038 758**

(73) Proprietor: **SMITHKLINE BECKMAN CORPORATION**
**P.O. Box 7929 1 Franklin Plaza**
**Philadelphia Pennsylvania 19101 (US)**

(72) Inventor: **Pfeiffer, Francis Richard**
**201 Sussex Drive**
**Cinnaminson New Jersey 08077 (US)**

(74) Representative: **Waters, David Martin, Dr. et al**
**Smith Kline & French Laboratories Ltd. Patent Department Mundells**
**Welwyn Garden City Hertfordshire AL7 1EY (GB)**

**EP 0 080 283 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention comprises a group of new chemical compounds which have biological activity whose structures have a tripeptide chain with two prolyl end units separated by a spacer unit. One of the prolyl units has an essential N-(acid functionalized alkyl)-substituent. The biological activity of these compounds is to increase renal blood flow and induce diuretic activity. As such, the compounds are useful for treating hypertension or for improving renal function.

K. T. Poroshin et al., Chemical Abstracts, *53* 21693h, described the use of carbobenzoxy-L-prolylglycyl-L-proline as an intermediate to prepare L-prolylglycyl-L-proline, however, this reference described neither the essential N-carboxyalkyl substituent nor the critical mid-unit both of which characterize the structures of these new compounds. Poroshin, also, described no biological activity for any of the compounds he prepared. The compounds of this invention, therefore, differ from those in the prior art by two structural parameters.

EPO application 12,401 describes certain carboxy-alkyl peptides which are angiotensin converting enzyme inhibitors. The EPO compounds differ from those of the present invention in mechanism of action as well as in chemical structure.. The N-carboxyalkyl substituents are not substituted on the prolyl unit of the EPO dipeptides.

As stated above, the group of new chemical compounds of this invention is characterized by having tripeptide structures which are propyl-spacer-prolines with an acid functionalized alkyl substituent at the free N-member of the prolyl ring. The structures, therefore, contain two acid groups, one of which is amphoteric.

Exemplary of the compounds of this invention are those represented by formula I:

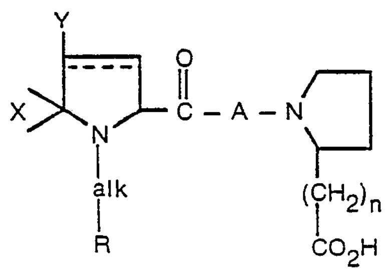

I

in which:

R is an acid group such as carboxy (—$CO_2H$), sulfo (—$SO_2OH$) or phosphono (—P(O)($OH_2$);
alk is a straight or branched alkylene chain of 1—5 carbons which is not an ethylidene (=CH—$CH_3$);
X is O or H,H;
Y is H or, when X is H,H, —OH;
- - - - is an optional carbon-carbon bond only when X and Y are H,H or H, respectively;
n is an integer of from 0—3, inclusive; and
A is a spacer amino acid unit in combined peptide form such as,

$CH_3$ / $CH_2$—$C_6H_5$
D- or L-Ala (—NH—CH—CO—); D- or L-Phe (—NH—CH—CO—);

$CH_3$ / $C_6H_5$
D- or L-β-methyl-β-Ala (—NH—CH—$CH_2$—CO—); D- or L-β-phenyl-β-Ala (—NH—CH—$CH_2$—CO);

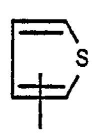

$C_6H_5$
D- or L-β-thienyl-Gly (—NH—CH—CO—) ; D- or L-phenyl-Gly (—NH—CH—CO—);

D- or L-β-Ala (—NH—$CH_2$—$CH_2$—CO—); γ-aminobutyric acid (—NH—$CH_2$—$CH_2$—$CH_2$—CO—);

$C_2H_5$ / $C_3H_7$
D- or L-2-aminobutyric acid (—NH—CH—CO—); D- or L-nor-Val (—NH—CH—CO—);

D- or L-nor-Leu (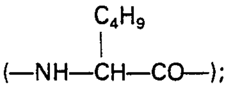); 3-amino-3-methylbutyric acid (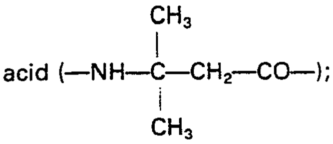); and 2-methyl-Ala (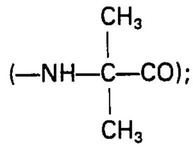); or N-methyl derivatives of said spacer units.

A subgeneric group of this invention comprises those compounds of formula I in which A is a substituted or unsubstituted β-Ala. A useful species is that of formula I in which R is carboxy, alk is methylene, X is H,H, Y is H, n is O and A is β-Ala. Preferably, the configuration of the amino acid units of each of the above groups of compounds will be L. The species, N-carboxymethyl-L-propyl-β-alanyl-L-proline sulfate, is an advantageous compound of this invention.

In the definition of the compounds of formula I above, the end unit in which X is O will be recognized as a pyroglutamyl ring, the unit in which Y is OH will be recognized as a hydroxy propyl ring and the unit in which a double bond is present will be recognized as a dehydropropyl ring. All these are, essentially, prolyl derivatives.

In the renal anesthetized dog screening protocol described hereafter, N-carboxymethyl-L-propyl-L-lysyl-L-proline, N-carboxymethyl-propyl-glycyl-L-proline and N-(1-carboxyethyl)-L-propyl-β-alanyl-L-proline were inactive as renal vasodilators in the primary test period of the screening protocol. This illustrates the critical nature of the spacer unit as well as of the N-alkyl substituent on the propyl end unit.

These compounds include the various pharmaceutically acceptable salt forms of the invention, such as those formed with nontoxic acids at the basic N-member of the N-carboxyalkylpropyl fragment or those formed having pharmaceutically acceptable cations associated with the anionic acid groups. The former include the sulfate, hydrochloride, phosphate, hydrobromide, ethanedisulfonate or methane-sulfonate. The latter include the sodium, potassium, calcium and similar salts with strong bases. The alkali metal salts are most useful as intermediates rather than end products although they may be used either way.

The salts are formed by reacting the compounds of formula I in a suitable solvent with an appropriate acid or base, using reaction conditions which will be readily apparent to those skilled in the art. Usually, an excess of the inorganic acid or base is reacted with the compound of this invention dissolved in water or in an appropriate organic solvent, such as aqueous ethanol. The compounds of this invention, often, form solvates, such as hydrates or lower alcoholates.

The acid addition salts, such as the sulfate, are most useful forms of the compounds of this invention as are the parent tripeptides.

Further, the diacids of formula I may be used in pro-drug forms such as an amide, a lower alkyl ester derivative of from 1—5 carbons in each of said alkyl groups or a benzyl ester.

These compounds are prepared by a synthetic sequence in which the key reaction is as follows:

A.

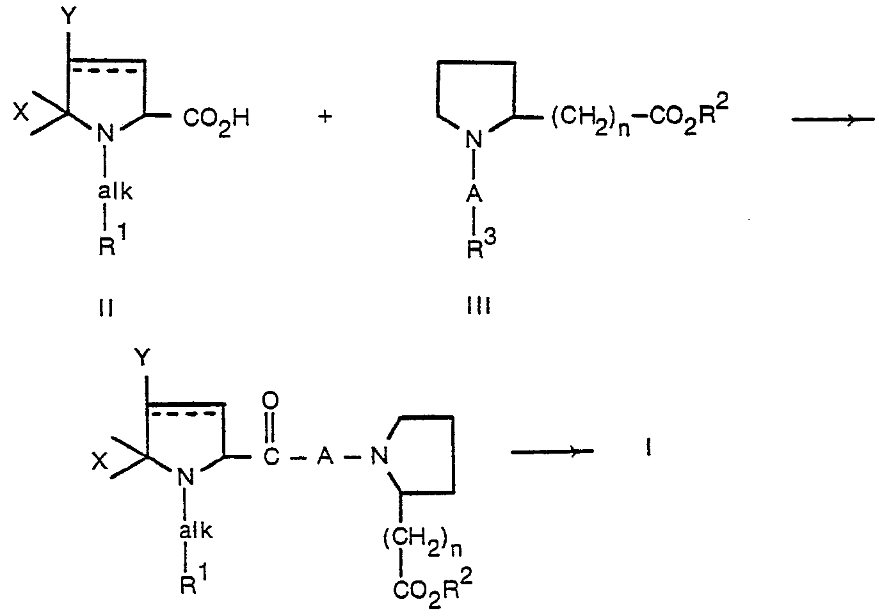

II III

IV

I

In sequence A, X, Y, ---- or n are as defined above. $R^1$ is a protected form of carboxy, sulfo or phosphono, such as a benzyl ester or lower alkyl ester thereof, $R^2$ is a carboxy protective group, such as benzyl or lower alkyl, and $R^3$ is the amino ($—NH_2$) or N-methylamino

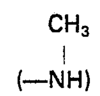

group of the spacer amino acid unit.

An N-(acid functionalized alkyl) proline (II) in a form which will protect the side chain acid group is reacted with a proline-containing dipeptide with its terminal acid group protected (III) to give the products of this invention in the form of the protected dicarboxylic acids (IV). In the formation of the amide bond during the reaction between the amino acid and the dipeptide starting materials, any reaction conditions of a peptide coupling reaction are used. Especially useful is the reaction of the amino acid unit (II) with the dipeptide (III) in the presence of an acylation promotor such as a carbodiimide. The reaction is carried out in an organic solvent, for example, tetrahydrofuran, dimethylacetamide or dimethylformamide using moderate temperatures until the reaction is complete. Room temperature for from 1—18 hours is used in many cases.

Other coupling methods used involve isoxazolium derivatives, 1-ethoxycarbonyl-2-ethoxy-1,2-dihydroquinoline, a phosphonium derivative, the N-carboxy anhydride derivatives or a N-hydroxysuccinimide ester derivative. More specific details of various coupling methods are described in "Peptide Synthesis", Bodanszky, Wiley (1976) Chapter Five.

The protecting ester groups are, then, removed by standard reactions which do not effect the amide bonds, such as by using catalytic hydrogenation for O-benzyl groups or by using controlled alkali hydrolysis, such as by using barium hydroxide.

The compounds are, also, prepared utilizing solid phase technology commonly used in preparing peptides.

The compounds of this invention have pharmacodynamic activity and, acting on the kidney, are useful pharmaceutical compounds. More specifically, they increase renal blood flow or decrease renal vascular resistance. Their effect in improving kidney function, often, appears to be cumulative. They, also, have diuretic activity. These compounds, therefore, are relatively long acting antihypertensive or kidney function improving agents. Surprisingly, the compounds of this invention have activity after either oral or parenteral administration.

The biological activity of the compounds of formula I was demonstrated by administering the compounds by infusion to anesthetized dogs measuring the mean arterial blood pressure, renal blood flow, renal vascular resistance and heart rate in the test procedure explained in detail in U.S. Patent No. 4,197,297. Generally speaking, the compounds gave a decreased renal vascular resistance and increased renal blood flow at doses ranging from 1/3 to 1/100 of that for dopamine in this test procedure. Representative specific results are included in the examples.

The compounds, alternatively, demonstrate biological activity in spontaneously hypertensive rats (SHR). In this test, male, Okamato-Aoki strain, spontaneously hypertensive rats, aged 16—19 weeks, are

fasted, then, the following afternoon, the first dose of test compound is adminstered, along with a 25 ml/kg p.o. load of normal saline. The animals are, then, placed in metabolism cages and urine collected for three hours for analysis. Indirect systolic blood pressure and heart rate are measured by a tail-cuff. An identical second dose of test compound is adminstered. After two hours, blood pressure and heart rate are again tested. The test compound is usually administered p.o. or i.p. in saline with 0.02% ascorbic acid. In this test, representative drugs of this invention demonstrated diuretic activity.

For example, (N-carboxymethyl)-L)propyl-β-alanyl-L-proline hemisulfate i.p. demonstrated a significant increase in $Na^{\oplus}$, $K^{\oplus}$ or urine excretion at 25 and 50 mg/kg i.p. It had a RVR—$ED_{15}$ of 12 µg/kg in the anesthetized renal dog protocol (dopamine was 3.5 µg/kg). It did not have angiotensin coverting enzyme inhibiting activity at 10 times its renally effective dose. This species, also, did not stimulate or antagonize the dopamine-sensitive adenylate cyclase system *in vitro,* nor was it active in the toad bladder anti-ADH assay. The compound demonstrated a weak bradycardic effect.

The new chemical compounds, described above, are incorporated into dosage unit forms such as capsules, tablets or injectable preparations which are useful for improving renal function, treating high blood pressure or inducing diuresis. The pharmaceutical carrier for the dosage units employed may be, for example, either a solid or liquid. Exemplary of solid carriers are lactose, terra alba, sucrose, talc, gelatin, agar, pectin, acacia, magnesium stearate or stearic acid. Exemplary of liquid carriers are syrup, peanut oil, olive oil or water. Similarly, the carrier or diluent may include any time delay material well known to the art, such as glyceryl monostearate of glyceryl distearate alone or with a wax. Such sustained release products as well as derivatives which may be gradually metabolized to the active parent can be employed to prolong the unique biological activity of the compounds of this invention if considered necessary.

A wide variety of pharmaceutical forms can be employed. Thus, if a solid carrier for oral administration is used the preparation can be tableted, placed in a hard gelatin capsule in powder, suppositories regular or sustained release pellet form, in the form of a troche or lozenge. The amount of solid carrier will vary widely but, preferably, will be from about 25 mg to about 1 g. If a liquid carrier is used, the preparation will be in the form of a syrup, emulsion, soft gelatin capsule, sterile injectable liquid such as an ampul, or an aqueous or nonaqueous liquid suspension.

The pharmaceutical preparations are made following the conventional techniques of the pharmaceutical chemist involving mixing, granulating and compressing when necessary for tablet forms, or mixing, filling, and dissolving the ingredients, as appropriate to give the desired oral or parenteral end products.

The doses of the present compounds in the pharmaceutical dosage unit will be an effective, nontoxic quantity selected from the range of 10—350 mg of active tripeptide, preferably 50—200 mg. The selected dose is administered to a patient in need of treatment for the one of the noted clinical conditions from 1—5 times daily, orally, by injection or by infusion. Parenteral administration which uses a lower dose chosen from the dose range, individually or combined, is preferred, however, oral administration, from 1—5 times a day at higher doses, is also used when convenient for the patient.

One skilled in the art will recognize that the compounds of this invention may exist in various configurations such as optical isomers or mixtures thereof. Isomeric compounds are easily prepared by substituting the amino acid of a selected configuration into the chemical reactions of the examples which illustrate this invention or by chromatographic separation of an isomeric mixture by high pressure liquid chromatography.

The following examples are intended to teach the preparation and use of the new compounds of this invention but not to limit its scope. All temperatures are expressed in degree Centigrade.

## Example 1

A mixture of 6.0 g (0.025 mm) of the benzyl ester of L-proline, 4.7 g (0.025 m) of N-*tert.*-butoxycarbonyl-L-alanine, 6.75 g (0.05 m) of 1-hydroxybenzotriazole, 3.2 ml (2.82 g, 0.025 m) of N-ethylmorpholine and 65 ml of dry tetrahydrofuran was cooled, filtered and added to a mixture of 5.15 g (0.025 m) of dicyclohexylcarbodiimide and 10 ml of tetrahydrofuran. The mixture was stirred at 0° for 30 minutes, then, at 25° for 18 hours. The reaction mixture was filtered. The filtrate was evaporated to give a residue which was dissolved in ethyl acetate. The solution was washed with 1 to 10% acetic acid, water, 5% sodium bicarbonate solution and brine, then, dried over anhydrous magnesium sulfate and concentrated to leave 9.8 g of crude N-*tert.*-butoxycarbonyl-L-alanyl-L-proline, benzyl ester (the *t*-boc).

A mixture of 2 g of the *t*-boc, 3.0 g of trifluoroacetic acid and 15 ml of dry methylene chloride was allowed to react at room temperature for 2 hours, then, evaporated under reduced pressure to give the crude trifluoroacetate salt. The salt was dissolved in ether and acidified with ethereal hydrogen chloride to give 1.15 g white solid L-alanyl-L-proline, benzyl ester hydrochloride, m.p. 162—164°.

About 500 mg of 10% palladium-on-charcoal, wet with water, was added to a solution of 16.5 g (0.06 m) of N-carbomethoxymethyl-L-proline benzyl ester [R. Adams, J. Am. Chem. Soc. *81* 5803 (1959)] and 50 ml of ethanol. After shaking under hydrogen on a low pressure hydrogenation apparatus for 2 hours, the mixture was filtered. The filtrate was evaporated. After ethanol was added and evaporated, the syrupy residue was dissolved in 1:1 toluene-ether and acidified with cold ethereal hydrogen chloride. The white solid resulting was separated and washed with ether to give 13.8 g of crude N-carbomethoxymethyl-L-proline hydrochloride, m.p. 191—193°.

A mixture of 2.23 g (0.01 m) of N-carbomethoxymethyl-L-proline hydrochloride, 3.13 g (0.01 mole) of L-alanyl-L-proline, benzyl ester hydrochloride, 2.70 g (0.02 m) of 1-hydroxybenzotriazole, 2.3 g (0.02 m) of N-ethylmorpholine and 40 ml of dry tetrahydrofuran was cooled in ice/water, then, reacted with 2.06 (0.01 m) of dicyclohexylcarbodiimide at room temperature for 17 hours. the solids were separated by filtration. The filtrate was diluted with ethyl acetate and washed 3 times with 50 ml of 1.5% acetic acid, water, 5% sodium bicarbonate solution and brine. The dried organic layer was evaporated to give 4.3 g of a yellow syrup. the syrup was taken over a silica gel column and eluted with methylene chloride, 1% methanol/methylene chloride and 1—1/2% methanol/methylene chloride to give 2.1 g (47%) of N-carbomethoxymethyl-L-prolyl-L-alanyl-proline, benzyl ester, a pro-drug of the desired end product.

This material, 2.1 g, in 30 ml of methanol was added to a mixture of 5.0 g of barium hydroxide hydrate in 40 ml of water. After stirring for 4 hours, carbon dioxide gas was passed through the mixture to precipitate barium carbonate. The colorless filtrate was evaporated to give a white solid, 1.25 g (78%), as the desired tripeptide product.

A sample (300 mg) was recrystallized from methanol/ethyl acetate to give a white solid, m.p. 270° (dec.) which is the barium salt.

The unpurified solid (0.9 g) was dissolved in water. The mixture was filtered. The filtration was adjusted to 3.5 pH with dilute sulfuric acid. The barium sulfate was separated by filtration. The filtrate was evaporated to give N-carboxymethyl-L-propyl-L-alanyl-L-proline sulfate, 190—196° (dec.).

Anal. Calcd. for $C_{15}H_{23}N_3O_6 \cdot 1/4\ H_2SO_4 \cdot 1/2\ H_2O$: C, 48.06; H, 6.59; N, 11.20. Found: C, 48.48, 48.21; H, 6.69, 6.78; N, 10.80, 10.74 $[\alpha]_D^{25}$ = (C,1, 1:1, $CH_3\ OH.H_2O$) −132.2°.

In the renal vasodilator activity test in 3 anesthetized dogs, the barium salt gave a 21.7% increase in renal blood flow and 15% decrease in renal vascular resistance at 30 µg/kg/min.

Example 2

A mixture of 6.0 g (0.025 m) of the benzyl ester of L-proline hydrochloride, 6.75 g (0.05 m) of 1-hydroxybenzotriazole, 3.2 ml (0.025 m) of N-ethylcarbonylphenylalanine, 5.15 g (0.025 m) of dicyclohexylcarbodiimide and 35 ml of dry tetrahydrofuran was stirred at 0° briefly, then, at 25° for 4 hours. The mother liquor was separated and evaporated. The residue was dissolved in ethyl acetate and dilute hydrochloric acid. The layers separated. The aqueous layer was extracted with ethyl acetate. The combined organic layers were washed with dilute hydrochloric acid, water, 5% sodium, bicarbonate solution and brine. The organic layer was dried and evaporated to give 10.3 g (91%) of N-*tert.*-butoxycarbonyl-L-phenylalanyl-L-proline, benzyl ester.

This material (7.0 g) was reacted with a solution of 10 ml of trifluoroacetic acid, 40 ml of dry methylene chloride and 4 ml of 1,3-dimethoxybenzene at 25° for 2 hours. Working up as described above gave 5.57 g of L-phenylalanyl-L-proline, benzyl ester, hydrochloride.

The benzyl ester (2.23 g, 0.01 m) was reacted with 3.89 g of N-carbomethoxymethyl-L-proline hydrochloride, 2.70 g (0.02 m) of 1-hydroxybenzotriazole, 4.0 ml of N-ethylmorpholine, 2.06 g (0.01 m) of dicyclohexylcarbodiimide and 40 ml of dry tetrahydrofuran at 25° for 17 hours, as described above, to give 2.9 g of N-carbomethoxymethyl-L-prolyl-L-proline, benzyl ester, purified over silica gel.

This diester pro-drug (1.1 g, 2.1 mm) was dissolved in 30 ml of methanol and reacted with a warm solution of 5.0 g of barium hydroxide in 40 ml of water. The mixture was stirred at 25° for 4 hours, then, excess carbon dioxide was added. The barium carbonate was removed by filtration using a filter aid. The filtrate was evaporated and the residue azeotroped with ethanol to give, as described above, 0.72 g (82%) of the barium salt and, then, 0.89 g of the hemisulfate salt of N-carboxymethyl-L-prolyl-L-phenylalanyl-L-proline, m.p. 145—148°.

Anal. Calcd. for $C_{21}H_{25}N_3O_6 \cdot Ba \cdot H_2O$: C, 51.15; H, 5.31; N, 8.52; Ba, 13.92. Found: C, 51.47, 51.64; H, 5.27, 5.50; N, 5.28, 5.28; Ba 12.60 $[\alpha]_D^{25}$ = (C,1, $H_2O$) −43.9°.

Anal. Calcd. for $C_{21}H_{27}N_3O_6 \cdot 1/2H_2SO_4$: C, 54.07, H, 6.04, N, 9.01. Found: C, 53.71, 53.64, H, 5.90, 5.94; N, 8.82, 8.91.

This sulfate increased renal blood flow at 30 and 300 µg/kg/min. The barium salt was not active within the parameters of anesthetized dog screening test.

Example 3

Using the methods described above, 7.6 g of N-*tert.*-butoxycarbonyl-β-alanyl-L-proline, benzyl ester was prepared by the described acylation using dicyclohexylcarbodiimide. The protective group was removed by using trifluoroacetic acid to give β-alanyl-L-proline, benzyl ester hydrochloride. This compound (6.39 g, 0.02 m) was condensed with 2.23 g (0.01 m) of N-carbomethoxymethylproline hydrochloride by reacting in the presence of 2.7 g (0.02 m) of 1-hydroxybenzotriazole, 12 ml of N-ethylmorpholine, 2.06 g (0.01 m) of dicyclohexylcarbodiimide in 50 ml of dry tetrahydrofuran at room temperature fo 17 hours. The product was 1.9 g of syrupy N-carbomethoxymethyl-L-prolyl-β-alanyl-L-proline, benzyl ester, the pro-drug precursor of the desired diacid.

The diester (1.75 g, 3.9 mm) was hydrolyzed using barium hydroxide and methanol, then, treated with sulfuric acid to give 1.2 g of a white solid, N-carboxymethyl-L-prolyl-β-alanyl-L-proline sulfate.

Anal. Calcd. for $C_{15}H_{23}N_3O_6 \cdot 1/2\ H_2SO_4$: C, 46.15; H, 6.20; N, 10.76. Found: C, 45.81; 46.14; H, 6.50, 6.52; N, 9.94, 10.22; $[\alpha]_D^{25}$ = (0.8, $H_2O$) −70.4°.

In the spontaneously hypertensive rat test (n=3) at 25 mg/kg i.p., the compound gave the following results in the diuretic naturetic assay: $Na^{\oplus}$ 676.04 (control 126.76); $K^{\oplus}$ 242.53 (85.12); Urine, ml/rat, 16(3). It also increased renal blood flow and decreased renal vascular resistance in two anesthetized dogs at 3, 30 and 300 μg/kg/min. In a secondary test in three dogs, this compound had an $ED_{15}$ of 12 μg/kg compared to 3.5 μg/kg for dopamine (i.v.).

Example 4

Using the same reactions described above, 7.23 g (0.03) of ethyl β-bromopropionate and 7.33 g (0.04 m) of proline benzyl ester hydrochloride gave N-(2-carbethoxy)-ethyl-L-proline, benzyl ester (4.6 g, 50.2%). This compound (4.6 g, 0.015 m) was hydrogenated over palladium-on-carbon to give 2.96 g (78%) of N-(2-carbethoxy)ethyl-L-proline, hydrochloride, m.p. 169—171°. This compound (24.2 g, 0.0096 m) was condensed with 2.25 g (0.0072 m) of L-alanyl-L-proline, benzyl ester hydrochloride in the dicyclohexylcarbodiimide procedure to give 0.8 g of N-(2-carbethoxy)ethyl-L-propyl-alanyl-L-proline, benzyl ester. The pro-drug precursor was hydrolyzed with barium hydroxide in aqueous methanol to give 0.498 g (64%) of N-(2-carboxyethyl)-L-prolyl-L-alanyl-L-proline, m.p. 133—136°.

Anal. Calcd. for $C_{16}H_{25}N_3O_6{\cdot}C_2H_5OH\ 1H_2O$; C, 51.54; H, 7.93; N, 10.02. Found: C, 51.95, 51.72; H, 7.12, 7.32; N, 10.19, 10.04. $[\alpha]_D^{25}$ = (1, $H_2O$) −147.3°.

This compound in 2 anesthetized dogs demonstrated an accumulative effect at 300 μg/kg/min dose of 34% increase in renal blood flow and 23% decrease in renal vascular resistance.

Example 5

D,L-β-Methyl-β-alanyl-L-proline, benzyl ester hydrochloride (5.95 g, 0.017 m, prepared as described in Example 1) was reacted with 4.41 g (0.015 m) of L-N-[5-(carbomethoxy)pentyl]proline, hydrochloride, which had been prepared by reacting ethyl-6-bromocaproate with proline, benzyl ester hydrochloride followed by catalytic hydrogenation to split the benzyl ester. The condensing agent was dicyclohexylcarbodiimide in the presence of 1-hydroxybenzotriazole and N-ethylmorpholine in tetrahydrofuran. The product was 1.5 g of syrupy N-5-carboethoxypentamethylenyl)-L-prolyl-D,L-β-methyl-β-alanyl-L-proline, benzyl ester, m/e at 530 is M + 1. Barium hydroxide hydrolysis of 0.5 g of the ester gave 0.2 g of the desired L-(N-5-carboxypentamethylenyl)prolyl-D,L-β-methyl-β-alanyl-L-proline, m.p. 80—84°; $[\alpha]_D^{25}$ = (C, 1, 1:1 methanol/water) −90.1°.

Anal. Calcd. for $C_{20}H_{33}N_3O_6{\cdot}1.25\ H_2O$: C, 55.35; H, 8.36; N, 9.68. Found: C, 55.49; H, 8.78; N, 9.05.

Example 6

Using the same methods described above, 12.5 g (0.76 m) of D,L-3-amino-3-phenylpropionic acid was reacted with 9.6 g (0.095 m) of triethylamine and 20.7 g (0.095 m) of di-*tert.*-butyldicarbonate in 250 ml of dimethylformamide at 25° for 17 hours to give 19.9 g (79%) of the white solid *t*-boc derivative, m.p. 115—117°. This material, 7.95 g (0.03 m) was condensed with 7.23 g (0.05 m) of L-proline, benzyl ester hydrochloride to give 13.6 g of the dipeptide. The protective group was removed by treatment with trifluoroacetic acid in methylene dichloride and 1,3-dimethoxybenzene to give, after anhydrous hydrogen chloride treatment, D,L-β-phenyl-β-alanyl-L-proline, benzyl ester, hydrochloride as a white solid. This material (10.1 g, 0.026 m) was condensed with 5.81 g (0.026 m) of L-N-carbomethoxymethylproline hydrochloride to give, after chromatography over silica gel with a methanol in methylene chloride gradient, 1.8 g of N-carbomethoxymethyl-L-prolyl-D,L-β-phenyl-β-alanyl-L-proline, benzyl ester. Hydrolysis of the diester with barium hydroxide followed by acidification with dilute sulfuric acid gave 0.99 of N-carboxymethyl-L-prolyl-D,L-β-phenyl-β-alanyl-L-proline, sulfate, m.p. 255—260°, $[\alpha]_D^{25}$ = (C,1, 1:1 methanol/water) −43.2°.

Anal. Calcd. for $C_{21}H^{25}N_3O_6{\cdot}0.25\ H_2SO_4{\cdot}H_2O$: C, 54.83; H, 6.46; N, 9.13. Found: C, 54.86; H, 6.43; N, 8.24.

Example 7

Using the same reactions described above, 12.5 g (0.12 m) of D,L-β-methyl-β-alanine (D,L-3-aminobutyric acid) was reacted with 12.12 g (0.15 m) of triethylamine and 32.7 (0.15 m) of di-*tert.*-butyldicarbonate in 300 ml of dimethylformamide to give 18.5 g (76%) of the white solid *t*-boc., m.p. 90—92°. This material (4.06 g, 0.02 m) was condensed with 4.82 g (0.02 m) of proline, benzyl ester hydrochloride using the dicyclohexylcarbodiimide reaction described above, at 25° for 17 hours, to give, after trifluoroacetic acid and anhydrous hydrogen chloride treatment, D,L-β-methyl-β-alanyl-L-proline, benzyl ester, hydrochloride. This material (3.5 g, 0.01 m) was condensed with 1.12 g (0.005 m) of N-carbomethoxymethylproline hydrochloride, as discussed above, to give N-carbomethoxymethyl-L-prolyl-D,L-β-methyl-β-alanyl-L-proline, benzyl ester. Barium hydroxide hydrolysis, then, sulfuric acid salt formation gave N-carboxymethyl-L-prolyl-D,L-β-methyl-β-alanyl-L-proline hemisulfate, m.p. 135° $[\alpha]_D^{25}$ = −63.3° (C,1, 50% aqueous methanol).

The diastereoisomeric mixture (S, S, S and S, R, S) was separated by application of the mixture in 9:1 water-methanol to a C—18 reverse phase high pressure liquid chromatographic column using 90:10:0.1 of water-methanol-trifluoroacetic acid as the eluent. Base line separation (α=1.29) allowed the separation of the S, S, S and S, R,S—isomers with optical rotations of $[\alpha]_D^{25}$ = −59.9° (C,1, 1:1 $CH_3OH{\cdot}H_2O$) and $[\alpha]_D^{25}$= −44.8° (C,1, 1:1, $CH_3OH{\cdot}H_2O$), respectively.

Substituting N-(2-carbethoxy)ethyl-L-proline, hydrochloride for its lower homologue in this procedure

gives N-(2-carboxy)ethyl-L-prolyl-D,L-β-methyl-β-alanyl-L-proline. Substituting N-(3-carbomethoxy)propyl-D,L-proline, hydrochloride (prepared as described in Example 4) into the procedure of Example 4 in place of N-(2-carbethoxy)-ethyl-L-proline hydrochloride gives N-carboxypropyl-D,L-prolyl-L-alanyl-L-proline as the base.

Example 8

A mixture of 10.0 g (0.0529 m) of N-*tert.*-butoxy-carbonyl-D-alanine, 12.75 g (0.0529 m) of L-proline, benzyl ester hydrochloride, 14.28 g (0.0106 m) of 1-hydroxybenzotriazole, 15 ml of N-methylmorpholine and 125 ml of dry tetrahydrofuran together with 10.9 g (0.0529 m) of dicyclo-hexycarbodiimide and 15 ml of dry dimethylformamide, reacted and worked up as described above, gave 20.4 g of crude N-*tert.*-butoxycarbonyl-D-alanyl-L-proline benzyl ester. Removal of the *t*-boc protective group, as described, gave D-alanyl-L-proline, benzyl ester hydrochloride, m.p. 140—142°. This material (8.3 g, 0.265 m) was condensed with 5.92 g (0.0265 m) of N-carbomethoxymethyl-L-proline-hydrochloride as described, to give 3.1 g of syrupy N-carbomethoxyethyl-L-prolyl-D-alanyl-L-proline, benzyl ester. Barium hydroxide treatment of 3.0 g (6.7 mm) of the above diester gave 2.1 g of N-carboxymethyl-L-prolyl-D-alanyl-L-proline; m.p. 145—148° $[\alpha]_D^{25} = -39.9°$ (C,1, 1:1 $CH_3OH \cdot H_2O$).

Similarly N-2-carboxyethyl-L-prolyl-D-alanyl-L-proline was prepared; m.p. 170°d. (softening at 142—145°) $[\alpha]_D^{25} = -67.9°$ (C,1, 1:1 $CH_3OH \cdot H_2O$).

Example 9

A mixture of 6.28 g (0.0332 m) of N-*tert.*-butoxycarbonyl-β-alanine, 5.0 g (0.0332 m) of L-proline amide hydrochloride, 8.96 g (0.0664 m) of 1-hydroxybenzotriazole, 12 ml of N-methylmorpholine, 75 ml of tetrahydrofuran, 40 ml of dimethylformamide and 6.84 g (0.0332 m) of dicyclohexylcarbodiimide was stirred at 25° for 17 hours. The solvents were evaporated *in vacuo* and the residue dissolved in ethyl acetate and water. The separated organic layer was washed with small volumes of dilute hydrochloric acid, brine, 5% bicarbonate and brine. The dried extract was concentrated to give 3.2 g of syrupy N-*tert.*-butoxycarbonyl-β-alanyl-L-proline amide. The methane chemical ionization mass spectrum was in agreement with the dipeptide structure.

This product was dissolved in 35 ml of dry methylene dichloride, cooled in ice water and 15 ml of trifluoroacetic acid was added. The solution was stirred at 0° for 15 minutes and at 25° for 3 hours. the solvents were evaporated *in vacuo* at 40° to give the syrupy trifluoroacetate salt of β-alanyl-L-proline amide. This compound (~0.01 m) was condensed with N-carbomethoxy-methyl-L-proline hydrochloride, as described, to give N-carbomethoxymethyl-L-prolyl-β-alanyl-L-proline amide. Barium hydroxide treatment gave N-carboxymethyl-L-prolyl-β-alanyl-L-proline amide.

Example 10

Substituting N-carbomethoxymethyl-L-dehydroproline, benzyl ester, prepared by the Adams alkylation method, for the N-carbomethoxymethyl-L-proline ester in Example 1 gives N-carboxymethyl-L-dehydroprolyl-L-alanyl-L-proline as the sulfate salt. Substituting in Example 1 N-carbomethoxymethyl-pyroglutamic acid, benzyl ester, prepared by the Adams alkylation method, gives N-carboxymethyl-pyroglutamyl-L-alanyl-L-proline. Substituting in Example 1 2-pyrrolidinylacetic acid, benzyl ester, for the benzyl ester of L-proline gives *N*-carboxymethyl-L-propyl-L-alanyl-2-pyrrolidinylacetic acid. Substituting the *t*-boc derivative of β-(2-thienyl)alanine (The Chemistry of Heterocyclic Compound Volume III, Hartough, page 262) for the alanine derivative of Example 1 gives N-carboxymethyl-L-propyl-D,L-β-(2-thienyl)-alanyl-L-proline. Substituting N-methylsulfomethylproline benzyl ester, prepared using the known methyl ester of chloromethanesulfonic acid in the Adams alkylation process, in Example 1 gives N-sulfomethyl-L-propyl-L-alanyl-L-proline. Using the known dimethyl ester of chloromethylphosphonic acid in the Adams alkylation and, then, substituting N-dimethylphosphonomethyl proline benzyl ester in Example 1 gives N-phosphonomethyl-L-prolyl-L-alanyl-L-proline.

**Claims for the Contracting States: BE CH DE FR GB IT LI LU NL SE**

1. A chemical compound of the structural formula:

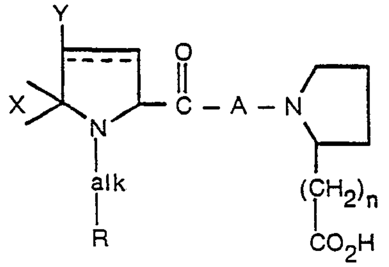

in which

R is carboxy, sulfo or phosphono;

alk is a straight or branched alkylene chain of 1—5 carbon which is not ethylidene;

X is O or H,H;

Y is H, and can also be —OH when X is H,H; and when X and Y are H,H and H respectively, - - - - - - is optionally a carbon-carbon bond;

n is an integer of from 0—3 inclusive; and

A is an amino acid residue derived from D- or L-Ala, D- or L-Phe, D- or L-β-methyl-β-Ala, D- or L-phenyl-Gly, D- or L-β-phenyl-β-Ala, D- or L-β-thienyl-Gly, D- or L-β-Ala, γ-aminobutyric acid; D- or L-2-aminobutyric acid, D- or L-nor-Val, D- or L-nor-Leu, 3-amino-3-methyl butyric acid, 2-methyl-Ala or a N-methyl derivative of said residues; or a pharmaceutically acceptable salt or prodrug derivative of said compound.

2. A compound according to claim 1 in which the configuration of the amino acid units is L.

3. A compound according to claim 2 in which R is carboxy, X is H,H, Y is hydrogen and n is 0.

4. A compound according to claim 3 in which A is a β-Ala.

5. A compound according to claim 1 being N-(2-carboxymethyl)-L-prolyl-β-alanyl-L-proline or a pharmaceutically acceptable salt thereof.

6. A compound according to claim 1 being N-(2-carboxymethyl)-L-prolyl-β-alanyl-L-proline.

7. A compound according to claim 1 being N-(2-carboxymethyl)-L-prolyl-L-alanyl-L-proline or a pharmaceutically acceptable salt thereof.

8. A compound according to claim 1 being N-carboxymethyl-L-prolyl-L-phenylalanyl-L-proline or a pharmaceutically acceptable salt thereof.

9. A compound according to claim 1 being N-carboxymethyl-L-prolyl-D,L-β-methyl-β-alanyl-L-proline or a pharmaceutically acceptable salt thereof.

10. A pharmaceutical composition effective for improving kidney function comprising a nontoxic, therapeutically effective quantity of a compound of claims 1, 2, 3, 4, 5, 6, 7, 8 or 9 combined with a carrier therefor.

11. A process for preparing a compound according to claim 1 which comprises reacting a compound of the structural formula:

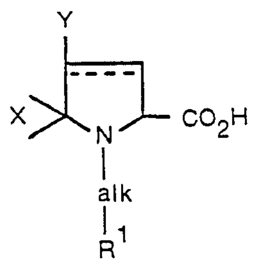

with a compound of the structural formula:

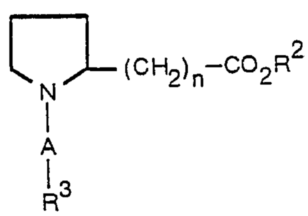

wherein A, X, Y, - - - - - , alk and n are as defined in claim 1, $R^1$ is protected carboxy, sulfo or phosphono; $R^2$ is a carboxy protective group; and $R^3$ is the amino or N-methylamino group which is part of A as defined in claim 1; removing any protective groups and optionally forming a pharmaceutically acceptable salt or prodrug derivative.

12. A compound according to any one of claims 1 to 9 for use in a method of therapeutic treatment.

13. A compound according to any one of claims 1 to 9 for use in increasing renal blood flow and inducing diuretic activity.

**Claims for the Contracting State: AT**

1. A process for preparing a tripeptide of the structural formula:

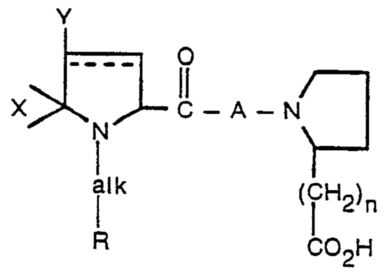

in which:

R is carboxy, sulfo or phosphono;

alk is a straight or branched alkylene chain of 1—5 carbon which is not ethylidene;

X is O or H,H;

Y is H, and can also be —OH when X is H,H;

and when X and Y are H,H and H respectively, ------ is optionally a carbon-carbon bond;

n is an integer of from 0—3 inclusive; and

A is an amino acid residue derived from D- or L-Ala, D- or L-Phe, D- or L-β-methyl-β-Ala, D- or L-phenyl-Gly, D- or L-β-phenyl-β-Ala, D- or L-β-thienyl-Gly, D or L-β-Ala, γ-aminobutyric acid; D- or L-2-aminobutyric acid, D- or L-nor-Val, D- or L-nor-Leu, 3-amino-3-methyl butyric acid, 2-methyl-Ala or a N-methyl derivative of said residues; or a pharmaceutically acceptable salt or prodrug derivative of said compound;

comprising reacting a compound of the structural formula:

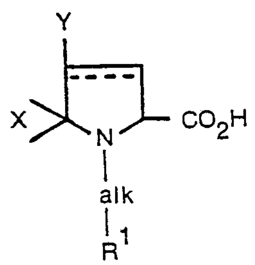

with a compound of the structural formula:

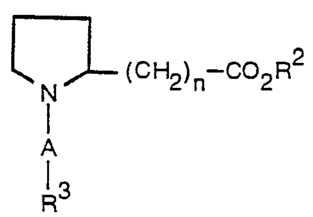

wherein A, X, Y, -----, alk and n are as hereinbefore defined, $R^1$ is protected carboxy, sulfo or phosphono; $R^2$ is a carboxy protective group; and $R^3$ is the amino or N-methylamino group which is part of A as hereinbefore defined; removing any protective groups and optionally forming a pharmaceutically acceptable salt or pro-drug derivative.

2. A process according to claim 1 in which the reaction is carried out in the presence of a carbodiimide.

3. A process according to claim 1 in which the reaction is carried out in the presence of dicyclohexylcarbodiimide.

4. A process according to claim 1 in which β-alanyl-L-proline as the benzyl ester hydrochloride is reacted with N-carbomethoxymethyl-L-proline hydrochloride to produce N-carboxymethyl-L-prolyl-β-alanyl-L-proline.

5. A process for preparing a pharmaceutical composition which comprises admixing a tripeptide as defined in claim 1 with a pharmaceutically acceptable carrier.

**Revendications pour les Etats contractants: BE CH DE FR GB IT LI LU NL SE**

1. Un composé chimique de la formule structurelle:

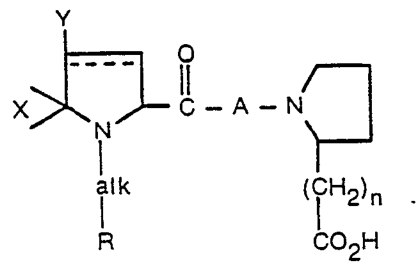

dans laquelle:

R est carboxy, sulfo ou phosphono;

alk est une chaîne alcoylène droite ou ramifiée de 1—5 carbones qu n'est pas éthylidène;

X est O ou H, H;

Y est H et peut aussi être —OH lorsque X est H, H; et lorsque X et Y sont H, H et H respectivement, ----- est facultativement un lien de carbone-carbone;

n est un nombre entier de 0—3 inclus; et

A est un résidu d'acide amino dérivé de D- ou L-Ala, D- ou L-Phe, D- ou L-béta-méthyl-béta-Ala, D- ou L-phényl-Gly, D- ou L-béta-phényl-béta-Ala, D- ou L-béta-thiényl-Gly, D- ou L-béta-Ala, γ-acide amino-butyrique; D- ou L-2-acide aminobutyrique, D- ou L-nor-Val, D- ou L-nor-Leu, 3-amino-3-méthyl acide butyrique, 2-méthyl-Ala ou un dérivé de N-méthyle des dits résidus; ou un sel pharmaceutiquement acceptable ou dérivé du pro-médicament du dit composé.

2. Un composé selon la revendication 2 dans laquelle la configuration des unités de l'acide amino est L.

3. Un composé selon la revendication 2 dans laquelle R est carboxy, X est H, H, Y est hydrogène et n est 0.

4. Un composé selon la revendication 3 dans laquelle A est un béta-Ala.

5. Un composé selon la revendication 1 qui est N-(2-carboxyméthyl)-L-prolyl-béta-alanyl-L-proline ou un sel du même pharmaceutiquement acceptable.

6. Un composé selon la revendication 1 qui est N-(2-carboxyméthyl)-L-prolyl-béta-alanyl-L-proline.

7. Un composé selon la revendication 1 qui est N-(2-carboxyéthyl)-L-prolyl-L-alanyl-L-proline ou un sel du même pharmaceutiquement acceptable.

8. Un composé selon la revendication 1 qui est N-carboxyméthyl-L-prolyl-L-phénylalanyl-L-proline ou un sel du même pharmaceutiquement acceptable.

9. Un composé selon la revendication 1 qui est N-carboxyméthyl-L-prolyl-D,L-béta-méthyl-béta-alanyl-L-proline ou un sel du même pharmaceutiquement acceptable.

10. Une composition pharmaceutique efficace pour améliorer la fonction des reins, comprenant une quantité non toxique, thérapeutiquement efficace d'un composé des revendications 1, 2, 3, 4, 5, 6, 7, 8 ou 9, combiné à un support convenable.

11. Un procédé pour la préparation d'un composé selon la revendication 1 qui comprend la réaction d'un composé de la formule structurelle:

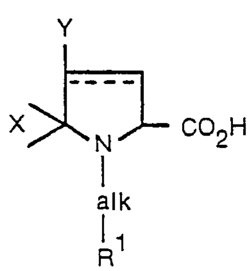

avec un composé de la formule structurelle:

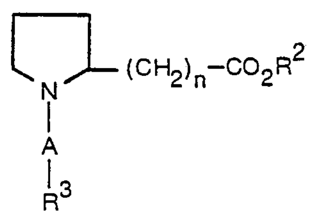

dans laquelle A, X, Y, -----, alk et n sont comme définis dans la revendication 1, $R^1$ est carboxy protégé, sulfo ou phosphono; $R^2$ est un groupe protecteur carboxy; et $R^3$ est le groupe amino ou N-méthylamino qui fait partie de A comme défini à la revendication 1; l'évacuation de tous les groupes protecteurs et la formation facultative d'un sel pharmaceutiquement acceptable ou d'un dérivé pro-médicament.

12. Un composé selon n'importe laquelle des revnedication de 1 à 9 à utiliser dans une méthode de traitement thérapeutique.

13. Un composé selon n'importe laquelle des revendications de 1 à 9 à utiliser dans l'accroissement du flot sanguin rénal et dans la sollicitation de l'activité diurétique.

**Revendications pour l'Etat contractant: AT**

1. Un procédé pour l préparation d'un tripeptide de la formule structurelle:

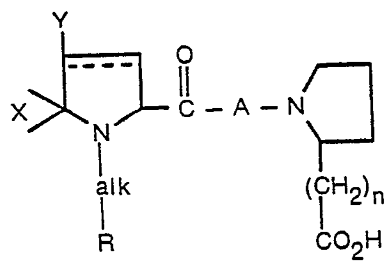

dans laquelle:

R est carboxy, sulfo ou phosphono;

alk est une chaîne alcoylène droite ou ramifiée de 1—5 carbones qui n'est pas éthylidène;

X est O ou H, H;

Y est H et peut aussi être —OH lorsque X est H, H; et lorsque X et Y sont H, H et H respectivement, ----- est facultativement un lien carbone-carbone;

n est un nombre entier de 0—3 inclusivement; et

A est un résidu d'acide amino dérivé de D- ou L-Ala, D- ou L-Phe, D- ou L-béta-méthyl-béta-Ala, D- ou L-phényl-Gly, D- ou L-béta-phényl-béta-Ala, D- ou L-béta-thiényl-Gly, D- ou L-béta-Ala, γ-acide amino-butyrique; D- ou L-2-acide aminobutyrique, D- ou L-nor-Val, D- ou L-nor-Leu, 3-amino-3-acide butyriqueméthyle, 2-méthyl-Ala ou un dérivé de N-méthyle desdits résidus; ou un sel pharmaceutiquement acceptable ou un dérivé pro-médicament du dit composé; comprenant la réaction d'un composé de la formule structurelle:

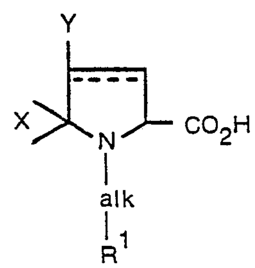

avec un composé de la formule structurelle:

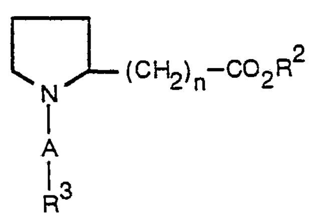

dans laquelle A, X, Y, -----, alk et n sont comme définis plus haut, $R^1$ est carboxy protégé, sulfo ou phosphono; $R^2$ est un groupe protecteur carboxy; et $R^3$ est le groupe amino ou N-méthylamino qui fait partie de A comme défini plus haut; l'évacuation de tous groupes protecteurs et la formation facultative d'un sel pharmaceutiquement acceptable ou d'un dérivé promédicament.

2. Un procédé selon la revendication 1 dans laquelle la réaction est effectuée en présence d'un carbodiimide.

3. Un procédé selon la revendication 1 dans laquelle la réaction est effectuée en présence d'un dicyclohexylcarbodiimide.

4. Un procédé selon la revendication 1 dans laquelle béta-alanyl-L-proline en tant que chlorhydrate d'ester de benzyl est mis en réaction avec le chlorhydrate de N-carbométhoxyméthyl-L-proline pour produire N-carboxyméthyl-L-prolyl-béta-alanyl-L-proline.

5. Un procédé pour la préparation d'une composition pharmaceutique qui consiste à ajouter tout en mélangeant un tripeptide, comme défini dans la revendication 1, avec un support pharmaceutiquement acceptable.

**Patentansprüche für die Vertragsstaaten BE CH DE FR GB IT LI LU NL SE**

1. Chemische Verbindung der Strukturformel:

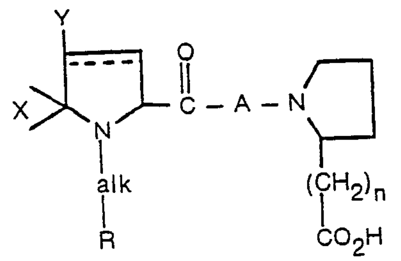

in der

R eine Carboxyl-, Sulfo- oder Phosphonogruppe ist

alk einen geradkettigen oder verzweigten Alkylenrest mit 1 bis 5 Kohlenstoffatomen bedeutet, der nicht die Äthylidengruppe ist;

X 0 oder H, H ist;

Y H ist, und auch —OH sein kann, wenn X H, H ist;

und wenn X und Y H, H bzw. H sind, ------ gegebenenfalls eine Kohlenstoff-Kohlenstoff-Bindung ist;

n eine ganze Zahl von 0 bis 3 einschließlich ist; und

A einen Aminosäurerest, abgeleitet von D- oder L-Ala, D- oder L-Phe, D- oder L-ß-Methyl-ß-Ala, D- oder L-Phenyl-Gly, D- oder L-ß-Phenyl-ß-Ala, D- oder 1-ß-Thienyl-Gly, D- oder L-ß-Ala, γ-Aminobuttersäure; D- oder L-2-Aminobuttersäure, D- oder L-nor-Val, D- oder L-nor-Leu, 3-Amino-3-methylbuttersäure, 2-Methyl-Ala oder ein N-Methylderivat dieser Reste bedeutet, oder ein pharmazeutisch verträgliches Salz oder ein Prodrug-Derivat der genannten Verbindung.

2. Verbindung nach Anspruch 1, in der die Konfiguration der Aminosäureeinheiten L ist.

3. Verbindung nach Anspruch 2, in der R eine Carboxylgruppe bedeutet, X H, H ist, Y Wasserstoff ist und n 0 ist.

4. Verbindung nach Anspruch 3, in der A ß-Ala ist.

5. Verbindung nach Anspruch 1, nämlich N-(2-Carboxymethyl)-L-prolyl-ß-alanyl-L-prolin oder ein pharmazeutisch verträgliches Salz davon.

6. Verbindung nach Anspruch 1, nämlich N-(2-Carboxymethyl)-L-prolyl-ß-alanyl-L-prolin.

7. Verbindung nach Anspruch 1, nämlich N-(2-Carboxyäthyl)-L-prolyl-L-alanyl-L-prolin oder ein pharmazeutisch verträgliches Salz davon.

8. Verbindung nach Anspruch 1, nämlich N-Carboxymethyl-L-prolyl-L-phenylalanyl-L-prolin oder ein pharmazeutisch verträgliches Salz davon.

9. Verbindung nach Anspruch 1, nämlich N-Carboxymethyl-L-prolyl-D,L-ß-methyl-ß-alanyl-L-prolin oder ein pharmazeutisch verträgliches Salz davon.

10. Zur Verbesserung der Nierenfunktion wirksames Arzneimittel, umfassend eine nicht-toxische, therapeutisch wirksame Menge einer Verbindung der Ansprüche 1, 2, 3, 4, 5, 6, 7, 8 oder 9, verbunden mit einem Träger dafür.

11. Verfahren zur Herstellung einer Verbindung nach Anspruch 1, gekennzeichnet durch die Umsetzung einer Verbindung der Strukturformel

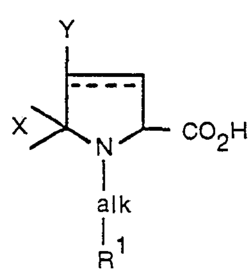

mit einer Verbindung der Strukturformel:

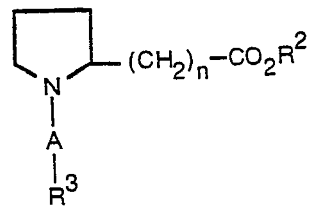

wobei A, X, Y, - - - - -, alk und n wie in Anspruch 1 definiert sind, $R^1$ eine geschützte Carboxyl-, Sulfo- oder Phosphonogruppe bedeutet; $R^2$ eine Carboxyschutzgruppe bedeutet; und $R^3$ die Amino- oder N-Methylaminogruppe darstellt, die Teil von A nach der Definition in Anspruch 1 ist; Abtrennung aller Schutzgruppen und gegebenenfalls Herstellung eines pharmazeutisch verträglichen Salzes oder Prodrug-Derivates.

12. Verbindung nach einem der Ansprüche 1 bis 9 zur Verwendung in einem Verfahren zur therapeutischen Behandlung.

13. Verbindung nach einem der Ansprüche 1 bis 9 zur Verwendung bei der Erhöhung des renalen Blutflusses und der Induktion von diuretischer Aktivität.

**Patentansprüche für den Vertragsstaat: AT**

1. Verfahren zur Herstellung eines Tripeptids der Strukturformel

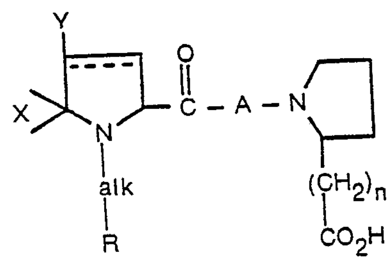

in der

R eine Carboxyl-, Sulfo- oder Phosphonogruppe ist

alk einen geradkettigen oder verzweigten Alkylenrest mit 1 bis 5 Kohlenstoffatomen bedeutet, der nicht die Äthylidengruppe ist;

X 0 oder H, H ist;

Y H ist, und auch —OH sein kann, wenn X H, H ist;

und wenn X und Y H, H bzw. H sind, - - - - - - gegebenenfalls eine Kohlenstoff-Kohlenstoff-Bindung ist;

n eine ganze Zahl von 0 bis 3 einschließlich ist; und

A einen Aminosäurerest, abgeleitet von D- oder L-Ala, D- oder L-Phe, D- oder L-ß-Methyl-ß-Ala, D- oder L-Phenyl-Gly, D- oder L-ß-Phenyl-ß-Ala, D- oder 1-ß-Thienyl-Gly, D- oder L-ß-Ala, γ-Aminobuttersäure; D- oder L-2-Aminobuttersäure, D- oder L-nor-Val, D- oder L-nor-Leu, 3-Amino-3-methylbuttersäure, 2-Methyl-Ala oder ein N-Methylderivat dieser Reste bedeutet, oder eines pharmazeutisch verträglichen Salzes oder Prodrug-Derivates der genannten Verbindung; gekennzeichnet durch Umsetzung einer Verbindung der Strukturformel:

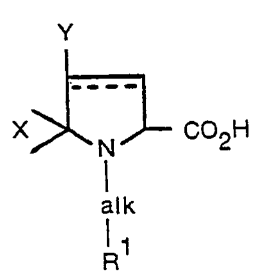

mit einer Verbindung der Strukturformel:

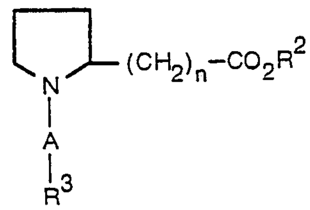

wobei A, X, Y, -----, alk und n wie in Anspruch 1 definiert sind, $R^2$ eine geschützte Carboxyl-, Sulfo- oder Phosphonogruppe bedeutet, $R^2$ eine Carboxyschutzgruppe bedeutet; und $R^3$ die Amino- oder N-Methylaminogruppe darstellt, die Teil von A nach der Definition in Anspruch 1 ist; Abtrennung aller Schutzgruppen und gegebenenfalls Herstellung eines pharmazeutisch verträglichen Salzes oder Prodrug-Derivates.

2. Verfahren nach Anspruch 1, bei dem die Umsetzung in Gegenwart eines Carbodiimids durchgeführt wird.

3. Verfahren nach Anspruch 1, bei dem die Umsetzung in Gegenwart von Dicyclohexylcarbodiimid durchgefühft wird.

4. Verfahren nach Anspruch 1, bei dem ß-Alanyl-L-prolin als Benzylesterhydrochlorid mit N-Carbomethoxymethyl-L-prolin-hydrochlorid zur Herstellung von N-Carboxymethyl-L-prolyl-ß-alanyl-L-prolin umgesetzt wird.

5. Verfahren zur Herstellung eines Arzneimittels, gekennzeichnet durch Vermischen eines Tripeptids gemäß Anspruch 1 mit einem pharmazeutisch verträglichen Träger.